# EUROPEAN PATENT APPLICATION

(11) **EP 4 806 996 A1**
(43) Date of publication of application: **16.09.2026**
(21) Application number: 24888095.7
(22) Date of filing: 08.11.2024
(51) Int. Cl.: C12N 9/42, C12N 9/24, C12N 15/09, C07K 16/00, C12N 15/00, A61K 39/395

(54) **NOVEL ENDO S2 MUTANT ENZYME**

(30) Priority: 10.11.2023 CN 202311501600; 31.10.2024 CN 202411548065
(71) Applicant: Shanghai Qilu Pharmaceutical Research and Development Centre Ltd., Shanghai 201203 (CN)
(72) Inventor: ZHAO, Yue, Shanghai 201203 (CN); WEN, Yunping, Shanghai 201203 (CN); FANG, Yan, Shanghai 201203 (CN); LI, Yaran, Shanghai 201203 (CN); LIU, Ziqun, Shanghai 201203 (CN); WANG, Hong, Shanghai 201203 (CN); QIN, Ken, Shanghai 201203 (CN); YANG, Yang, Shanghai 201203 (CN); YING, Hua, Shanghai 201203 (CN); TAO, Weikang, Shanghai 201203 (CN)
(74) Representative: Bandpay & Greuter
(86) International application number: PCT/CN2024/130895
(87) International publication number: WO 2025/098484

(57) **Abstract**

Provided is a novel Endo S2 mutant enzyme, which exhibits significantly enhanced transglycosylation activity and retains the majority of the hydrolytic activity. The enzyme can be used for polypeptides or proteins having N-glycans, such as glyco-engineering of antibodies, to perform sugar chain reconstruction.

## Description

The present application claims priority to Chinese Patent Application No. CN2023115016001, filed with the China National Intellectual Property Administration on November 10, 2023, entitled "NOVEL ENDO S2 MUTANT ENZYME"; and Chinese Patent Application No. CN202411548065X, filed with the China National Intellectual Property Administration on October 31, 2024, entitled "NOVEL ENDO S2 MUTANT ENZYME", the entire contents of which are incorporated herein by reference.

### TECHNICAL FIELD

The present disclosure relates to the field of carbohydrate chemistry and enzymology, and in particular to sugar chain engineering of polypeptides or proteins using a novel recombinant Endo S2 mutant enzyme, the engineered proteins such as antibodies being useful for site-specific glycoconjugation of antibody-drug conjugates.

### BACKGROUND

Antibody-Drug Conjugates (ADCs) use linkers to attach cytotoxins to antibodies, thereby achieving targeted transport and enrichment of the cytotoxins and precise killing of tumor cells by virtue of the specific targeting of antibodies to the tumor-associated antigen and the efficient loading capacity of the linked cytotoxins. Therefore, ADCs have lower side effects, broader therapeutic effects, and higher therapeutic indexes than conventional chemotherapeutic drugs, and have become one of the fastest-growing classes of drugs in recent years. A conventional ADC conjugation method utilizes lysine (Lys) or cysteine (Cys) which are abundant in antibodies and have good exposure to link cytotoxins. This random conjugation approach can lead to heterogeneity of the ADC, i.e., heterogeneity of the cytotoxic conjugation sites and the number of conjugations, which negatively impacts parameters such as pharmacokinetics, tolerability, efficacy, and the like. Subsequently developed site-specific conjugation techniques, such as specific amino acid modification technique (THIOMAB technique), non-natural amino acid insertion technique, glycosite-specific conjugation technique, and enzymatic conjugation technique, each have their own advantages and disadvantages.

A typical IgG antibody consists of two light chains and two heavy chains, four of which constitute three distinct domains, including two variable Fab domains (used to recognize and bind antigens) and one constant Fc domain (which binds to FcγR on the surface of receptor cells, mediating the interaction between the antibody and host cells). Almost all homodimers of therapeutic antibodies contain an N-glycan modification at the asparagine residue at position 297 (N297) in the Fc domain. This conserved glycosylation site can serve as a conjugation site for cytotoxins, and based on this, a glycosite-specific conjugation technique has been developed. The advantage of the glycosite-specific conjugation technique is that the N297 glycan site in Fc domain is distant from the Fab domain, which avoids the risk of reduced antibody binding to the antigen after conjugation. At the same time, the conjugation method does not require design or modification of the amino acid sequence of the antibody, and thus reducing the risk of immunogenicity.

Endoglycosidase, also known as endo β-N-acetamidoglucosidase, is a glycosidase that acts on the β-1,4-glycosidic bond. In recent years, endoglycosidase has gradually become the focus of research in the field of antibody glycoengineering and have been found to exhibit glycosyltransferase activity, making them useful in ADC glycosite-specific conjugation techniques. Endoglycosidases are derived from a variety of sources, including Endo S from Streptococcus pyogenes, Endo F1, Endo F2, Endo F3 from Flavobacterium meningosepticum, Endo S2 from serotype M49 Streptococcus pyogenes, and the like. Among them, Endo S2 exhibits broader substrate specificity, and capable of recognizing and acting on complex glycans (Endo S can not), high-mannose glycans (Endo S can not), complex multiple antennary glycans (Endo F1 can not), core non-fucosylated glycans (Endo F3 can not), and core fucosylated glycans. Therefore, Endo S2 holds significant potential in the field of preparing ADC in the glycosite-specific conjugation. However, Endo S2 belongs to the glycoside hydrolase 18 (GH18) family, which primarily exhibits glycosidic hydrolytic activity with lower transglycosylation activity, making it unsuitable for ADC production. Therefore, there is an urgent need for an Endo S2 mutant, which exhibits significantly enhanced transglycosylation activity and retains the majority of the hydrolytic activity, to facilitate the preparation of antibody-drug conjugate (ADC) drugs via one-step process for antibody glycosylation remodeling and site-specific glycoconjugation

### SUMMARY OF THE INVENTION

The present disclosure provides a recombinant Endo S2 mutant enzyme, which exhibits enhanced transglycosylation activity and retains the majority of the hydrolytic activity. The enzyme is capable of glyco-engineering polypeptides or proteins containing N-glycan, including glycoform hydrolysis and sugar chain remodeling. Therefore, the enzyme is suitable for one-step site-specific glycoconjugation of antibodies or Fc fragments thereof, such as for the synthesis of ADC drugs or antibody-containing detection drugs. The prepared ADCs possess the function of delivering therapeutic agents such as cytotoxins, exhibiting enhanced cytotoxicity against target cells.

The recombinant Endo S2 mutant enzyme of the present disclosure is derived from the endo β-N-acetamidoglucosidase (Endo S2) of the serotype M49 *Streptococcus pyogenes* NZ131 strain, the wild-type enzyme sequence of which is set forth in SEQ ID NO: 1. According to the description in the literature (Tiezheng Li et al, Glycosynthase mutants of endoglycosidase S2 show potent transglycosylation activity and remarkably relaxed substrate specificity for antibody glycosylation remodeling, 2018-12-22), Endo S2 can satisfy the hydrolytic and transglycosylation activities on the basis of retaining the continuous amino acids from position 38 to position 819 of SEQ ID NO: 1. That is, positions 38 to 819 of SEQ ID NO: 1 are the known active fragment of Endo S2, and various truncated fragments of the enzyme that comprise positions 38 to 819 of SEQ ID NO: 1 and still have hydrolytic and transglycosylation activities are within the scope of the present disclosure..

The Endo S2 mutant enzyme of the present disclosure preferably employs site-directed mutagenesis to have mutations at one or two amino acid positions selected from Y70, F106, G140, R176, D179, Q250, Y252, S285, E288, Y339, preferably at R176 and/or D179, in the sequence of SEQ ID NO: 1, or in an amino acid sequence with at least 95% sequence identity to SEQ ID NO: 1, or in a sequence comprising contiguous amino acids at positions 38 to 819 of SEQ ID NO: 1.

In some preferred embodiments, the Endo S2 mutant enzyme of the present disclosure has mutations at one or two amino acid positions selected from Y70, F106, G140, R176, D179, Q250, Y252, S285, E288, Y339, preferably at R176 and/or D179, in an amino acid sequence with at least 95%, at least 96%, at least 97%, at least 98%, or at least 99% sequence identity to SEQ ID NO: 1.

Still further, in some preferred embodiments, the mutation is selected from at least one of Y70F, F106W, F106Y, G140N, R176Y, D179A, Q250Y, Q250F, Y252F, S285D, S285A, E288F, E288R, Y339F, R176Y/D179A, more preferably, the mutation is R176Y and/or D179A. Domains and fragments comprising any of the Endo S2 mutant enzymes above are included in the present disclosure, wherein any such domains and fragments may be fused with other proteins, including, but not limited to, CPD, Fc, MBP, and the like. The protein of the mutant may be modified or truncated, including, but not limited to, at the N-terminus and the C-terminus. In some embodiments, the sequence preceding at least one amino acid between M1 and E37 of the mutant may be cleaved and deleted from the N-terminus, and/or the sequence following at least one amino acid between Y820 and D843 of the mutant may be cleaved and deleted from the C-terminus.

Based on the function of the Endo S2 mutant enzyme disclosed above, it can be used for glyco-engineering of the N-glycan-containing polypeptide or protein. More specifically, it can be used to remodel the glycoform of an antibody, or reconstruct the sugar chain of a glycoprotein, or prepare detection drugs or ADCs drug comprising the antibody.

The present disclosure provides a method for sugar chain remodeling of an N-glycan-containing polypeptide or protein using a one-step process, comprising the following steps:
(a) introducing the Endo S2 mutant enzyme of the present disclosure;
(b) introducing an N-glycan-containing polypeptide or protein as a substrate;
(c) providing a sugar chain linker donor; and
(d) using the Endo S2 mutant enzyme to perform glycosidic hydrolysis on the N-glycan-containing polypeptide or protein and transfer the sugar chain linker donor to the glycosidic hydrolysis product, thereby providing a new sugar chain-engineered polypeptide or protein; and
the one-step process does not include a purification step for the glycosidic hydrolysis product.

The one-step process does use the Endo S2 mutant enzyme disclosed in the present invention, which possesses both glycosidic hydrolysis and transglycosylation activity. In the same reaction step, both deglycosylation and transglycosylation are carried out simultaneously, thus eliminating the need for the subsequent purification step of the glycosidic hydrolysis product. This makes the process more convenient and efficient for industrial applications.

Meanwhile, the present disclosure also provides a method for sugar chain remodeling of an N-glycan-containing polypeptide or protein using a two-step process in which two enzymes are introduced to perform glycosidic hydrolysis and new glycosyl transfer, respectively, wherein the Endo S2 mutant enzyme of the present disclosure is used in at least one step of glycosidic hydrolysis or new glycosyl transfer, the method comprising the following steps:
(a) introducing the Endo S2 mutant enzyme of the present disclosure or other suitable endoglycosidases;
(b) introducing an N-glycan-containing polypeptide or protein as a substrate, and performing glycosidic hydrolysis under the action of the enzyme in step (a), thereby providing a glycosidic hydrolysis product as an acceptor for subsequent new glycosyl transfer;
(c) providing a sugar chain linker donor; and
(d) transferring the sugar chain linker donor to the acceptor using the Endo S2 mutant enzyme or another suitable glycosyltransferase to produce a new sugar chain-engineered polypeptide or protein;
optionally, a purification step for the glycosidic hydrolysis product may also be included between steps (b) and (c).

The two-step process begins with a deglycosylation reaction, where hydrolysis is performed by an endoglycosidase, in which the Endo S2 mutant enzyme of the present disclosure may be selected, or other suitable enzymes may be selected. Next, the transglycosylation reaction is carried out using a transglycosylase, in which the Endo S2 mutant enzyme of the present disclosure may be selected, or other suitable enzymes may be selected. The Endo S2 mutant enzyme is used in at least one step of glycosidic hydrolysis or new glycosyl transfer. Also, whether the glycosidic hydrolysis product is purified after the deglycosylation step can be determined as needed.

Further, the N-glycan-containing polypeptide or protein includes, but is not limited to, naturally occurring antibodies, recombinant antibodies, Fc fragments of antibodies, or sialoglycopeptides.

Further, the other suitable endoglycosidases include, but are not limited to, the following wild-type enzymes and mutants thereof: Endo S, Endo S2, Endo F1, Endo F2, Endo F3, Endo A, Endo D, Endo M.

Further, the other suitable glycosyltransferases include, but are not limited to, the following wild-type enzymes and mutants thereof: Endo S, Endo S2, Endo F1, Endo F2, Endo F3, Endo A, Endo D, Endo M.

Still further, the sugar chain linker donor includes, but is not limited to, natural N-glycan oxazoline or synthetic glycan oxazoline. Preferably, a disaccharide, trisaccharide, tetrasaccharide, pentasaccharide, hexasaccharide, heptasaccharide, octasaccharide, nonasaccharide, decasaccharide, or undecasaccharide oxazoline and specific derivatives thereof are used as donors for transglycosylation, with a disaccharide oxazoline being particularly preferred. The natural N-glycan oxazoline includes complex glycan oxazoline, high-mannose glycan oxazoline, or hybrid oxazoline. In some embodiments, the selective derivatization types of sugar chain linkers include, but are not limited to, glycosyl fluorides, glycosyl azide modifications or arylglycans, and the like, with their tag portions including, but not limited to, therapeutic drugs, toxins, fluorescent probes, biotin, lipids, antigenic fragments and analogs thereof, PEGs, genes and analogs thereof, RNAs and analogs thereof, DNAs and analogs thereof, polypeptides, proteins and analogs thereof, enzymes, enzyme substrates, enzyme inhibitors, enzyme modulators, or antibody fragments. In some embodiments, the sugar chain linker can also be modified using additional modification methods, which may be azidation, aldehydation, alkynylation, sulfhydrylation, hydroxylation, carboxylation, phosphorylation, sialylation, albumination, farnesylation, or modification of conjugation to a polymer.

Further, the glycosidic hydrolysis product has N-acetylglucosamine or core fucosylated N-acetylglucosamine remaining on the peptide chain, serving as a reaction site for the next transglycosylation reaction, follwing glycosidic hydrolysis of the N-glycan-containing polypeptide or protein.

Still further, the present disclosure also provides an antibody generated using the sugar chain engineering method and ADC drugs thereof.

In some embodiments, the present disclosure provides a polynucleotide encoding the Endo S2 mutant enzyme described in the present disclosure.

In some embodiments, the present disclosure provides a vector comprising the polynucleotide described in the present disclosure.

In some embodiments, the present disclosure provides a host cell transformed with the vector described in the present disclosure.

### BRIEF DESCRIPTION OF THE DRAWINGS

FIG. 1 is a schematic diagram of the recombinant plasmid for fusion expression of Endo S2 with CPD protein and His-tag.
FIG. 2 shows the ESI-MS analysis of the deglycosylation treatment of monoclonal antibody 1. (A) ESI-MS of the heavy chain of monoclonal antibody 1 (after deconvolution); (B) ESI-MS of the heavy chain of the deglycosylated monoclonal antibody 1 (after deconvolution).
FIG. 3 shows the results of detection of glycosidic hydrolytic activity of wild-type Endo S2 and its mutants.
FIG. 4 shows a structure of an azide-modified disaccharide linker oxazoline.
FIG. 5 shows the ESI-MS analysis of transglycosylation treatment of core fucosylated and non-fucosylated GlcNAc-monoclonal antibody 1. (A) ESI-MS of the heavy chain of GlcNAc-monoclonal antibody 1 (after deconvolution); (B) ESI-MS of the heavy chain of the transglycosylated monoclonal antibody 1 (after deconvolution).
FIG. 6 shows the results of detection of transglycosylation activity of wild-type Endo S2 and its mutants.
FIG. 7 shows the results of detection of glycosidic hydrolysis and transglycosylation activity of wild-type Endo S2 and its preferred mutants on monoclonal antibody 2.
FIG. 8 shows the accumulation data of transglycosylation products of monoclonal antibody 1 by the preferred Endo S2 mutants.
FIG. 9 shows the results of the transglycosylation system reaction of monoclonal antibody 1 by the preferred Endo S2 mutants for 20 h.

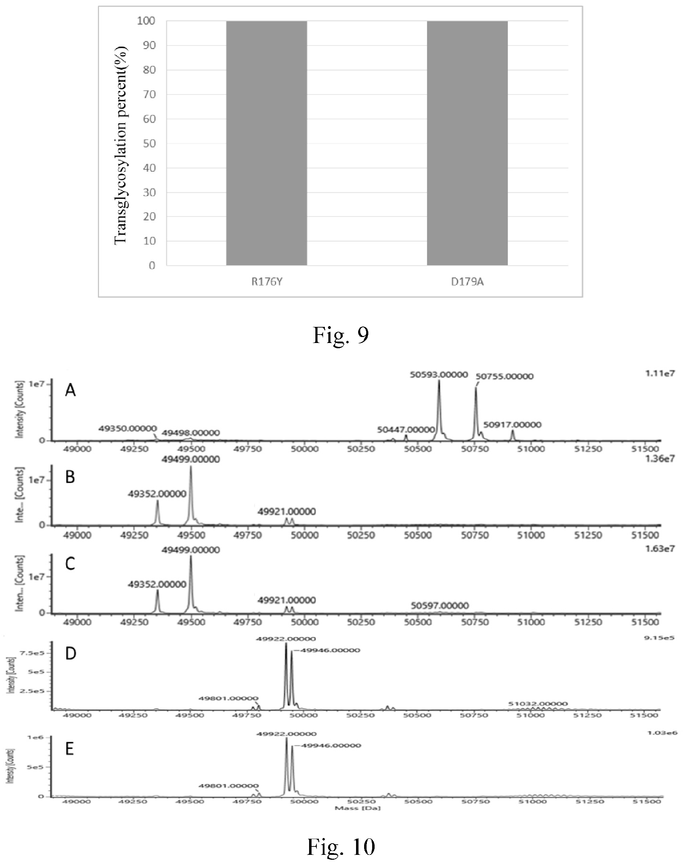

FIG. 10 shows the results of direct glycoform remodeling of intact antibody 1 by preferred Endo S2 mutants. (A) control group, ESI-MS of the heavy chain of intact monoclonal antibody 1 (after deconvolution); (B) ESI-MS of heavy chain of monoclonal antibody 1 after treatment with mutant T138Q (after deconvolution); (C) ESI-MS of heavy chain of monoclonal antibody 1 after treatment with mutant D184M (after deconvolution); (D) ESI-MS of heavy chain of monoclonal antibody 1 after treatment with mutant R176Y (after deconvolution); (E) ESI-MS of heavy chain of monoclonal antibody 1 after treatment with mutant D179A (after deconvolution).

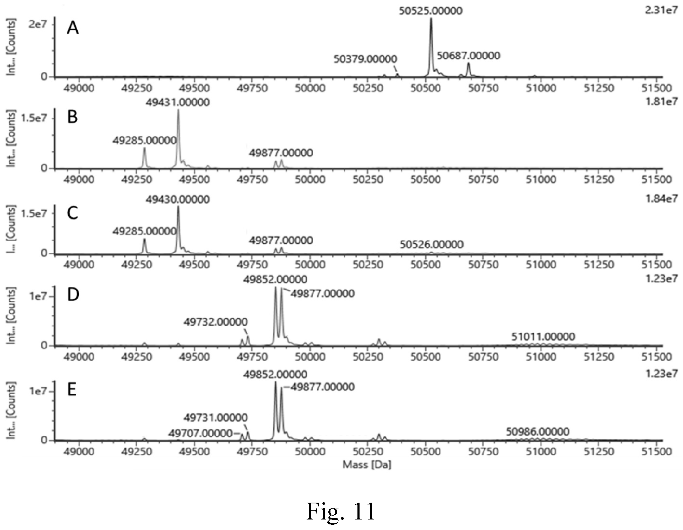

FIG. 11 shows the results of direct glycoform remodeling of intact antibody 2 by preferred Endo S2 mutants. (A) control group, ESI-MS of the heavy chain of intact monoclonal antibody 2 (after deconvolution); (B) ESI-MS of heavy chain of monoclonal antibody 2 after treatment with mutant T138Q (after deconvolution); (C) ESI-MS of the heavy chain of monoclonal antibody 2 after treatment with mutant D184M (after deconvolution); (D) ESI-MS of the heavy chain of monoclonal antibody 2 after treatment with mutant R176Y (after deconvolution); (E) ESI-MS of heavy chain of monoclonal antibody 2 after treatment with mutant D179A (after deconvolution).

### DETAILED DESCRIPTION OF THE INVENTION

Unless otherwise defined, the techniques used in this disclosure are conventional techniques in the fields of immunology, molecular biology, microbiology, cellular biology, genetic engineering, and protein engineering, and the academic terms used in this disclosure have the same meaning as commonly understood by those skilled in the art described in this disclosure. All publications and patent documents mentioned in this disclosure may be considered as references to those skilled in the art.

The three-letter and single-letter codes for amino acid residues used herein are as described in J. Biol. Chem, 243, p3558 (1968).

The mutant enzymes of the present disclosure are obtained using recombinant DNA techniques and the terms "mutant" or "mutant enzyme" are used interchangeably to refer to a mutated recombinant enzyme, which remains a protein molecule in its chemical nature and has sequence characteristics distinct from those of the wild-type enzyme.

Annotations of amino acids contained in the molecules of the present disclosure follow the standard practice in the field, with the position of the mutation indicated by the single-letter symbol of the wild-type amino acid and its number, for example, Arg at position 176 is referred to as "R176". The mutation is indicated by the single-letter symbol of the wild-type amino acid, its number, and the single-letter symbol of the mutated amino acid, for example, the mutation in which Arg at position 176 is replaced by Tyr is referred to as "R176Y". Additionally, if the mutant has multiple mutations, the multiple mutations are separated by the delimiter "/".

In the present disclosure, Endo S2 mutant R176 is a mutant consisting of a sequence in which Arg at position 176 of wild-type Endo S2, as set forth in SEQ ID NO: 1, is substituted by another natural amino acid. Endo S2 mutant D179 is a mutant consisting of a sequence in which Asp at position 179 of wild-type Endo S2, as set forth in SEQ ID NO: 1, is substituted by another natural amino acid. Endo S2 mutant R176/D179 is a mutant consisting of a sequence in which Arg at position 176 of wild-type Endo S2, as set forth in SEQ ID NO: 1, is substituted by another natural amino acid, and Asp at position 179 is substituted by another natural amino acid.

In the present disclosure, Endo S2 mutant R176Y is a mutant consisting of a sequence in which Arg at position 176 of wild-type Endo S2, as set forth in SEQ ID NO: 1, is substituted by Tyr. Endo S2 mutant D179A is a mutant consisting of a sequence in which Asp at position 179 of wild-type Endo S2, as set forth in SEQ ID NO: 1, is substituted by Ala. Endo S2 mutant R176/D179 is a mutant consisting of a sequence in which Arg at position 176 of wild-type Endo S2, as set forth in SEQ ID NO: 1, is substituted by Tyr, and Asp at position 179 is substituted by Ala.

The mutants of the present disclosure do not need to have a full-length sequence, and as long as they retain the regions critical for the transglycosylation activity of EndoS2, they all fall within the scope of the present disclosure. The known active fragment of Endo S2 is the amino acid sequence from positions 38 to 819 of SEQ ID NO: 1. On this basis, recombinant proteins comprising the fragment and suitably adding, for example, a signal peptide sequence, a purification tag (e.g. His-tag), a linker sequence (e.g. GGGS), or other functional components at the N-terminus or the C-terminus to form a new fusion protein without affecting the performance of the Endo S2 activity, all fall within the scope of the present disclosure.

In the amino acid sequences of the mutants of the present disclosure, one to several amino acids may be substituted, deleted, inserted, and/or added at positions other than the positions of the necessary mutations described below, as long as the enzyme activity is not affected, with an amino acid sequence having at least 95% sequence identity to SEQ ID NO: 1 being preferred. Any position can be selected for such amino acid changes, as long as it does not affect enzymatic activity, although the position is preferably one outside amino acid positions 38 to 819 of SEQ ID NO: 1. In the present disclosure, the term "several" refers to 20 or fewer, preferably 10 or fewer, further preferably 5 or fewer, and most preferably 4, 3, 2, or 1.

As used herein, "identity" refers to the percentage of amino acid residues in a candidate sequence that are identical to those in a reference sequence, after aligning the sequences (and introducing gaps, if necessary) to achieve the maximum percent sequence identity, and not considering any conservative substitutions as part of the sequence identity. Sequence alignment for determinationamino acid sequence identity can be achieved by various methods in the art, for example, using publicly available computer software such as BLAST, BLAST-2, ALIGN, or MEGALIGN (DNASTAR) software. Those skilled in the art can determine appropriate parameters for measuring alignment, including any algorithms needed to achieve maximal alignment over the full length of the sequences being compared.

When used in this disclosure, a reference without a specific quantity may mean one or more. As used in the claims of the present disclosure, when used in conjunction with the word "comprise", a reference without a specific quantity may mean one or more. When used in this disclosure, "another" may refer to at least a second or more.

As used herein, "sugar" refers to an oxidized or unoxidized carbohydrate-containing molecule including, but not limited to, monosaccharides, disaccharides, trisaccharides, oligosaccharides, or polysaccharides, including such as N-acetylglucosamine (L-isomer or D-isomer), mannose, galactose, N-acetylneuraminic acid (sialic acid), glucose, fructose, fucose, sorbose, rhamnose, mannoheptulose, N-acetylgalactosamine, dihydroxyacetone, xylose, xylulose, arabinose, glyceraldehyde, sucrose, lactose, maltose, trehalose, cellobiose, or any combination thereof. Sugars also refer to such molecules produced naturally, recombinantly, synthetically, and/or semi-synthetically.

As used herein, a "sugar chain linker" for glycosyl conjugation as an activated donor molecule may be a synthetic glycan oxazoline, such as an oligosaccharide with an activated reducing terminus, preferably an oligosaccharide molecule structured with an oxazoline; it may also be a natural N-glycan oxazoline. Sugar chain linkers may undergo chemical modification, such as by introducing functional groups through azidation, alkynylation, aldehydation, sulfhydrylation, or other methods, to better facilitate the loading of cytotoxins and the production of ADC-conjugated antibodies.

As used herein, "antibody," "monoclonal antibody," or "IgG" refers to a molecule containing an antigen-binding site that specifically binds to an antigen or an Fc region that binds to a cellular receptor, and these terms may be used interchangeably.

As used herein, "highly pure" refers to the target protein being seperarted from those contaminants that accompany it in its natural state or those contaminants generated or used during the process of obtaining the protein of interest. Typically, after gel electrophoresis, the gray scale of the bands of the target protein constitutes at least 90% of the total gray scale of all the bands, and the target protein is highly pure. Preferably, in certain embodiments, the gray scale of the bands of the target protein constitutes at least 95% of the total gray scale of all bands, most preferably at least 98%.

As used herein, "the majority of the hydrolytic activity" means that the glycosidic hydrolysis activity of the Endo S2 mutant is measured to be at least 50, preferably at least 60%, more preferably at least 70%, and most preferably at least 80% of that of the wild-type Endo S2 under the same conditions, with the glycosidic hydrolytic activity of the wild-type Endo S2 being defined as 100%.

Core fucosylated and non-fucosylated glycoproteins are important molecular classes that play a key role in many biological events such as tumor metastasis, cell adhesion, pathogen infection, and immune responses. Natural and recombinant fucosylated and non-fucosylated glycoproteins are generally produced as mixtures of glycoforms that differ only in the structure of the side chain oligosaccharides.

Core fucosylated and non-fucosylated derivatized sugar chain conjugation antibodies are produced according to the methods described herein. The functional groups introduced during derivatization include, but are not limited to, azide groups, aldehyde groups, alkyne groups, and sulfhydryl groups, which can be introduced into cytotoxins via chemical reactions to generate ADC-conjugated antibodies.

The present disclosure further provides a recombinant gene having the EndoS2 mutant as described above, a gene construct such as a plasmid or an expression vector comprising the recombinant gene, a host cell transformed with the gene construct, and a method for producing the mutant of the present disclosure which includes the step of collecting the mutant of the present disclosure from the host cell culture. The recombinant gene, gene construct, host cell, and the like may be prepared based on the amino acid sequences of the mutants of the present disclosure according to known genetic engineering techniques.

Host cells transformed by introducing genes encoding the mutants of the present disclosure can be cultured under conditions suitable for the species of the cells (cells commonly used for protein production, such as animal cells, plant cells, Escherichia coli, yeast, and the like, may be suitably selected), and mutants of the present disclosure may be collected from the culture. The collection of mutants is performed by appropriately combining conventional purification techniques based on the physical properties of the protein. To facilitate collection, gene constructs can be designed to express mutants with tag peptide, such as GST, the tag peptide is pre-linked to the mutant to allow the mutant to be collected using the affinity between the mutant and the tag peptide. The tag peptide may be removed after purification, but if it does not affect the enzymatic activity of the mutant, a mutant with an attached tag peptide can be used in reactions such as sugar reconstitution. Mutants of the present disclosure include amino acid sequences containing tag peptides attached thereto.

The following non-limiting Examples further illustrate the features and advantages of the present disclosure.

### EXAMPLES

### Materials and methods

Monoclonal antibody 1 and monoclonal antibody 2 used in the present disclosure were produced by the applicant. The azide-modified disaccharide linker LacNAc oxazoline (N3-LacNAc-Oxa) was purchased from Wuhan GLYCOGENE Pharmaceutical Co., Ltd., and the remaining reagents, unless otherwise specified, were conventional reagents produced by Merck, Sigma, Sinopharm, and others. The fillers, columns, and instruments used in the present disclosure include Cytiva Ni Sepharose Excel fillers, Cytive HiLoad^{™} 26/600 Superdex^{™} 200pg chromatographic columns, Acquity I-Class/RDa (Waters) liquid chromatography mass spectrometer, and ACCQUITY UPLC BEH PROTEIN C4 columns (Waters, 1.7 µm, 2.1 mm×50 mm).

### Site-directed Mutagenesis, Expression and Purification of Recombinant Endo S2 Wild-type and Mutant Enzymes

By PCR amplification, the cDNA encoding the Endo S2 amino acid sequence derived from the serotype M49 *Streptococcus pyogenes* NZ131 strain was fused with the cDNA encoding the cysteine protease domain (CPD) of the Vibrio cholerae MARTX toxin, and connected with a His-tag protein (10×His) at the C-terminus, and then together inserted between the SalI and NotI sites of the multicloning site region of the pET-22b (+) plasmid vector. The mutated amino acid codon was introduced using mutation-containing primers, and recombinant plasmids containing wild-type Endo S2 and its mutant nucleic acid sequence were produced by Escherichia coli DH5α strain, then transformed into Escherichia coli BL21 (DE3) cells. Expression of the EndoS2-CPD-10×His fusion protein (hereinafter referred to as Endo S2 fusion protein) was achieved by adding 0.25 mM isopropyl-beta-D-thiogalactopyranoside (IPTG) and culturing overnight at 20°C. The cells were collected by centrifugation, resuspended in lysis buffer (20 mM PB, 500 mM NaCl, pH 7.5), and then disrupted using an ultrasonic cell pulverizer (Wuxi Jieruian Instrument Equipment Co., Ltd.). After centrifugation, the cell lysate supernatant was collected, and the Endo S2 fusion protein was purified using Ni Sepharose Excel resin (Cytiva). It was then concentrated using a Amicon centrifugal filter (30 kDa, Millipore) and further purified by size exclusion chromatography with HiLoad^{™} 26/600 Superdex^{™} 200pg columns (Cytiva). The fractions containing the Endo S2 fusion protein were concentrated using an Amicon centrifugal filter (30 kDa, Millipore) and stored in storage buffer (20 mM PB, pH 7.5). Protein purity was analyzed by sodium dodecyl sulfate-polyacrylamide gel electrophoresis (SDS-PAGE) electrophoresis and gel imager (Gel Doc EZ Imager, Bio-RAD), using Image Lab scanning software, and the protein concentration was quantified using a spectrophotometer (Nano-300).

### Liquid Chromatography Mass Spectrometry (LC-ESI-MS) of IgG

LC-MS analysis was performed on Acquity I-Class/RDa (Waters). To analyze antibody heavy chains, IgG antibodies were treated with 20 mM TCEP and heated at 37°C for 30 minutes, then analyzed using ACCQUITY UPLC BEH PROTEIN C4 columns (1.7 µm, 2.1 mm×50 mm) with a 20%-50% linear gradient of MeCN containing 0.1% formic acid at a flow rate of 0.3 mL/min over 15 minutes. The raw data was deconvoluted using UNIFI (Waters).

### Preparation of GlcNAc-Monoclonal Antibody 1 and GlcNAc-Monoclonal Antibody 2

Intact monoclonal antibody 1 was dissolved in 20 mM phosphate, 150 mM NaCl, pH 8.0 buffer (10 mg/mL), and wild-type Endo S2 was added at a final concentration of 0.4 mg/mL, followed by incubation at 37°C for 3 hours. LC-MS analysis revealed complete cleavage of the N-glycan on the heavy chain, and the deglycosylated monoclonal antibody 1 was then purified by protein A chromatography. LC-MS data: m/z: 49354±2 Da (deconvolution data) corresponds to the heavy chain of non-fucosylated monoclonal antibody 1 (i.e., GlcNAc-monoclonal antibody 1); m/z: 49500±2 Da (deconvolution data) corresponds to the heavy chain of core fucosylated monoclonal antibody 1 (i.e., Fucα1,6-GlcNAc-monoclonal antibody 1).

GlcNAc-monoclonal antibody 2 was prepared in the same way as above. LC-MS data: m/z: 49285±2 Da (deconvolution data) corresponds to the heavy chain of non-fucosylated monoclonal antibody 2 (i.e., GlcNAc-monoclonal antibody 2); m/z: 49431±2 Da (deconvolution data) corresponds to the heavy chain of core fucosylated monoclonal antibody 2 (i.e., Fucα1,6-GlcNAc-monoclonal antibody 2).

### Glycosidic Hydrolytic activity Assay of Wild-type Endo S2 and Its Mutants

The glycosidic hydrolytic activity of wild-type Endo S2 and its mutants was determined at 37°C in a 20 mM phosphate buffer (pH 7.5) system, using 10 mg/mL intact monoclonal antibodies (e.g. monoclonal antibody 1 or monoclonal antibody 2) as substrates. The reaction was terminated by adding 0.1% formic acid to the mixture at a final concentration, and the product was then analyzed using reduced LC-MS. After deconvolution of the heavy chain ESI-MS raw data and integration of the corresponding MS peaks, the relative amounts of the substrate (monoclonal antibody 1) and the glycosidic hydrolysis product (deglycosylated monoclonal antibody 1) were quantified to calculate the change in the glycosidic hydrolytic activity of wild-type Endo S2 and its mutants.

### Transglycosylation Activity Assay of Endo S2 Wild-type and Its Mutants

10 mg/mL of deglycosylated monoclonal antibody (e.g. GlcNAc-monoclonal antibody 1 or GlcNAc-monoclonal antibody 2 containing core fucosylated and non-fucosylated GlcNAc-monoclonal antibody 1 or GlcNAc-monoclonal antibody 2), N3-LacNAc-Oxa (3.33 mM, 50 equivalents) were incubated with an appropriate amount of wild-type Endo S2 or its mutant in a 30°C, 20 mM phosphate buffer (pH 7.5) system, terminated by adding formic acid to a final concentration of 0.1%, and analyzed by reduced LC-MS.

### Accumulation of Transglycosylation Products of Endo S2 Mutants

Deglycosylated monoclonal antibody 1 (10 mg/mL, containing core fucosylated and non-fucosylated GlcNAc-monoclonal antibody 1), N3-LacNAc-Oxa (3.33 mM, 50 equivalents), and 200 nM Endo S2 mutants were mixed in a 30°C, 20 mM phosphate buffer (pH 7.5) system. At a series of time points, aliquots of the reaction mixture were taken. The reaction was terminated by adding formic acid to a final concentration of 0.1%, and analyzed by reduced LC-MS.

### Direct Transglycosylation of Endo S2 Mutants towards an Intact Antibody

N3-LacNAc-Oxa, intact monoclonal antibody (e.g. monoclonal antibody 1 or monoclonal antibody 2), and Endo S2 mutants were added to 20 mM phosphate buffer (pH 7.5) at final concentrations of 3.33 mM, 10 mg/mL and 0.2 mg/mL, respectively. After incubation at 30°C for 3 h, the reaction was terminated by adding formic acid to a final concentration of 0.1%. A control group without endoglycosidase was simultaneously set up to eliminate interference. The above samples were analyzed by reducing LC-MS.

### Example 1: Preparation of Recombinant Proteins of Wild-type Endo S2 and Endo S2 Mutants

Endo-β-N-acetamidoglucosidase (ENGase) (EC 3.2.1.96) is primarily classified into the GH85 and GH18 families, both of which follow a substantially consistent catalytic mechanism. The GH85 endoglycosidase includes Endo A and Endo M, while the GH18 endoglycosidase includes Endo S, as well as Endo F1, Endo F2, and Endo F3 from *Flavobacterium meningosepticum*, and Endo S2 from serotype M49 *Streptococcus pyogenes*. These GH18 endoglycosidases are widely recognized for their efficient hydrolysis of the asparagine-linked bibranched glycan at position 297 of IgG antibodies, resulting in the production of GlcNAc-Ig, with Endo S2 being particularly notable for its broad substrate specificity. These enzymes possess both glycosidic hydrolytic activity and transglycosylation activity, and therefore can serve as potentially advantageous enzymes for site-specific glycoconjugation techniques. Referring to the catalytic mechanism of homologous enzymes Endo S, Endo F1, Endo F2, and Endo F3, it is inferred that the catalytic process of Endo S2 also involves the substrate- assisted mechanism for the formation of oxazolinium ion intermediates. That is, aspartic acid (Asp) at position 184 of Endo S2 acts as a general acid to protonate the glycoside oxygenation, while the 2-acetamide group of GlcNAc acts as a nucleophile to replace the leaving group at the anomeric center, leading to the formation of an oxazoline ion intermediate. Subsequently, the catalytic carboxylate residue of glutamic acid (Glu) at position 186 in the side chain acts as a general base to activate a water molecule or glycosyl donor. Finally, the oxazoline intermediate reacts with the activated donor to catalyze glycoside hydrolysis or transglycosylation. The substrate binding pattern of Endo S2 has also been demonstrated by crystal structure, including crystal structure with high-mannose glycans (PDB ID: 6MDV) and crystal structure with complex diantennary glycan (PDB ID: 6MDS).

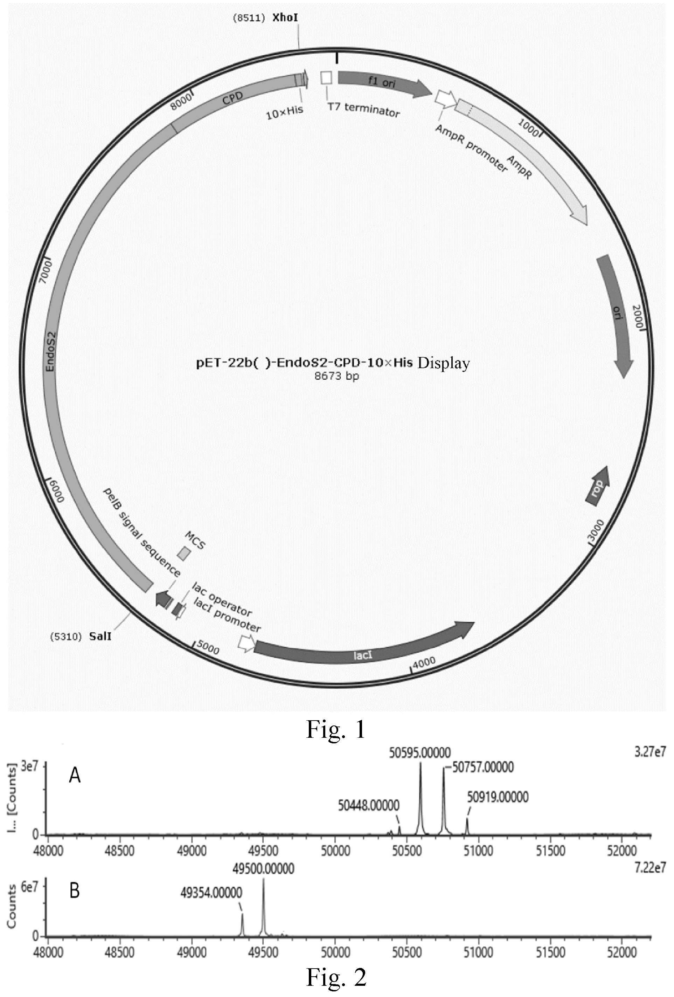

Based on the catalytic mechanism and substrate binding pattern described above, to explore transglycosylation of Endo S2, the present disclosure selects site-directed mutagenesis at positions Y70, F106, G140, R176, D179, Q250, Y252, S285, E288, Y339, including but not limited to Y70F, F106W, F106Y, G140N, R176Y, D179A, Q250Y, Q250F, Y252F, S285D, S285A, E288F, E288R, Y339F, and R176Y/D179A, with residues surrounding the catalytic active site and residues interacting with the substrate serving as entry points. The positions described above correspond to the sequence of SEQ ID NO: 1. The wild-type Endo S2 and its mutants were recombinantly expressed in *E. coli* by fusing the CPD protein at the C-terminus and adding a His-tag protein (see FIG. 1). The recombinant protein was referred to as the Endo S2 fusion protein, which can be purified by Ni Sepharose Excel affinity resin, and the fraction containing the target protein can be further purified using a HiLoadTM 26/600 SuperdexTM 200pg chromatography column to yield highly pure Endo S2 fusion protein.

### Example 2: Detection of Glycosidic Hydrolytic activity of Wild-type Endo S2 and Its Mutants

Monoclonal antibody 1 was used as a substrate to detect the glycosidic hydrolytic activity of wild-type Endo S2 and its mutants. The major Fc glycans of monoclonal antibody 1 are core fucosylated, double-branched complex oligosaccharides carrying 0, 1, or 2 galactose moieties, designated as G0F glycoform, G1F glycoform, and G2F glycoform, respectively. Monoclonal antibody 1 was deglycosylated using a wild-type Endo S2 fusion protein, and the deglycosylation process and results were detected by reduced LC-MS to obtain a fully deglycosylated monoclonal antibody 1. As shown in FIG. 2, the ESI-MS deconvolution data for the monoclonal antibody 1 heavy chain in FIG. 2A show 4 main different m/z substances, namely 50448±2, 50595±2, 50757±2, and 50919±2, which correspond to the G0 (core non-fucosylated and carrying 0 galactose), G0F, G1F and G2F glycoforms of the heavy chain, respectively. FIG. 2B is a graph showing the results of deglycosylation of monoclonal antibody 1, where the deconvolution data of ESI-MS of the heavy chain reveal two substances at 49354±2 Da and 49500±2 Da, which match the GlcNAc glycoform and fucosylated glycoform, respectively. The above results confirm the excellent glycosidic hydrolytic activity of wild-type Endo S2 against intact IgG, suggesting that it can be utilized in the first step of antibody site-specific glycoconjugation, namely the deglycosylation treatment of the antibody.

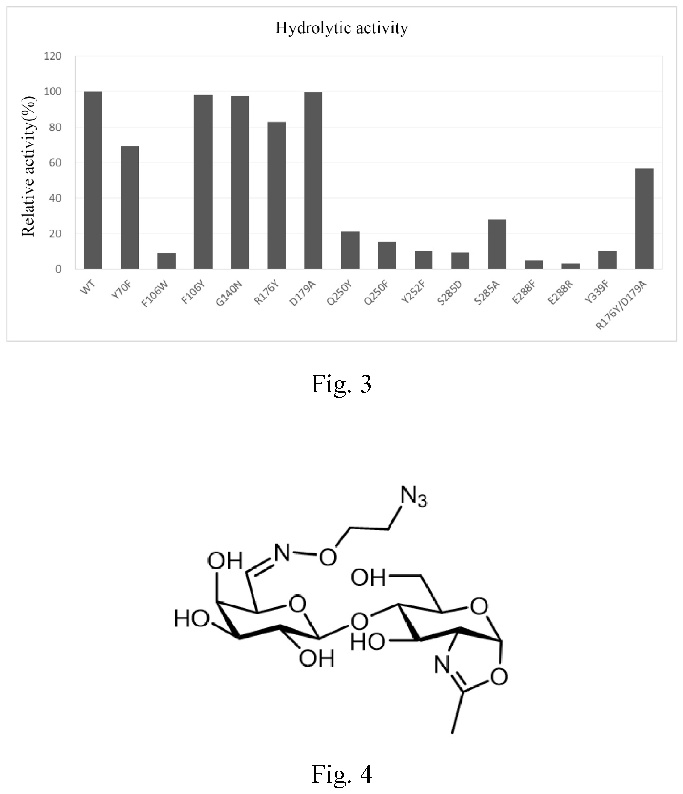

The glycosidic hydrolytic activity of wild-type Endo S2 and its mutants was determined using intact monoclonal antibody 1 as a substrate (intact monoclonal antibody 1 containing core fucosylation and non-fucosylation). As shown in FIG. 3, mutants Y70F, F106Y, G140N, R176Y, D179A, and R176Y/D179A retain most of the glycosidic hydrolytic activity compared to the wild-type Endo S2, and can also be used for the preparation of deglycosylated antibodies. In contrast, mutants F106W, Q250Y, Q250F, Y252F, S285D, S285A, E288F, E288R, and Y339F exhibit significantly reduced glycosidic hydrolytic activity, especially E288F and E288R, which almost lose all glycosidic hydrolytic activity.

### Example 3: Transglycosylation Activity Assay of Wild-type Endo S2 and Its Mutants

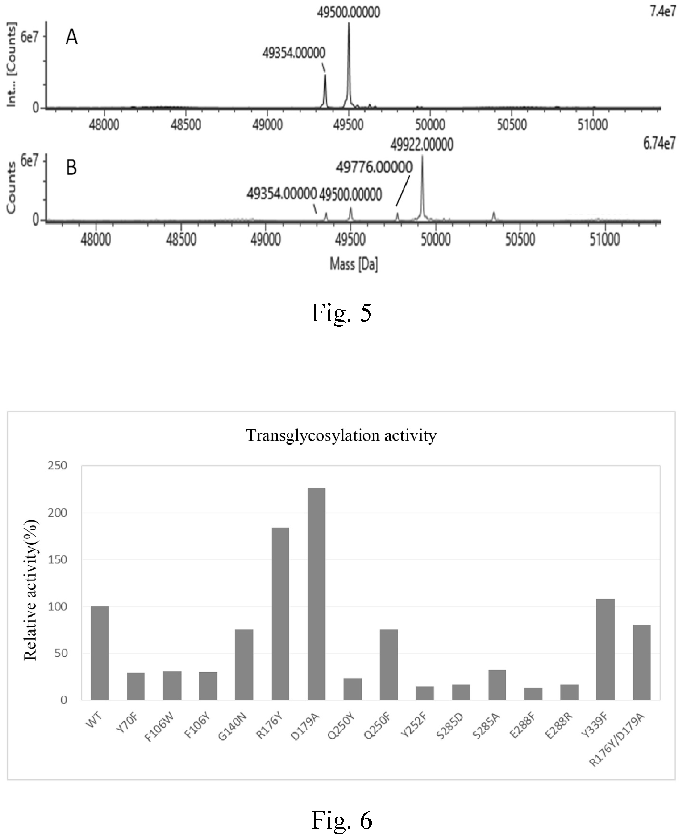

Transglycosylation activity of wild-type Endo S2 and its mutants was measured using core fucosylated and non-fucosylated GlcNAc-monoclonal antibody 1 as acceptors and azido-modified LacNAc disaccharide linker oxazoline (N3-LacNAc-Oxa, the structure of which is shown in FIG. 4) as donors. The wild-type Endo S2 fusion protein transglycosylation data analysis is shown in FIG. 5. FIG. 5A is the result of deglycosylated monoclonal antibody 1, with the deconvolution data of ESI-MS of the heavy chain showing two substances at 49354±2 Da and 49500±2 Da, corresponding to the GlcNAc glycoform and fucosylated glycoform, respectively. The ESI-MS data in FIG. 5B shows two additional substances at 49776±2 Da and 49922±2 Da, and these results matched well with the molecular weights after transglycosylation ligation with N3-LacNAc-Oxa (following azide degradation and protonation under acidic conditions).

The results of transglycosylation activity are shown in FIG. 6. The mutants R176Y and D179A exhibited significantly increased transglycosylation activity, while the transglycosylation activity of the mutants G140N, Q250F, Y339F, and R176Y/D179A was not significantly different from that of the wild-type, and the mutants Y70F, F106W, F106Y, Q250Y, Y252F, S285D, S285A, E288F, and E288R lost most of the transglycosylation activity. In combination with the results of the glycosidic hydrolytic activity assay in Example 2, it was surprisingly found that the two mutants, R176Y and D179A, retained most of the glycosidic hydrolytic activity while having significantly enhanced transglycosylation activity.

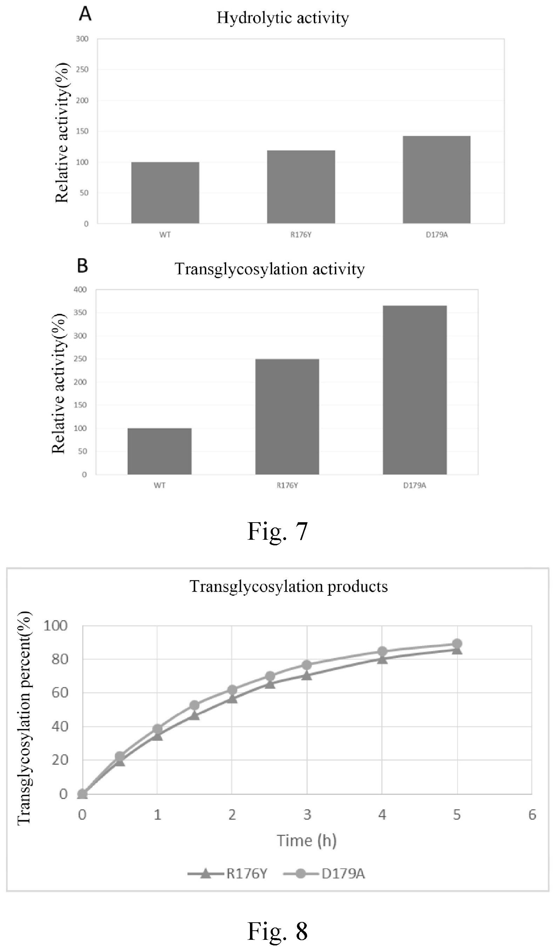

Meanwhile, the present disclosure also determines the glycosidic hydrolytic activity and transglycosylation activity of the preferred mutants R176Y and D179A towards monoclonal antibody 2, as shown in FIG. 7. FIG. 7A shows the glycosidic hydrolytic activity of the preferred mutants, with the hydrolytic activity of R176Y being comparable to that of the wild-type, and the hydrolytic activity of D179A being slightly increased, indicating that the mutants R176Y and D179A retain at least the majority of the hydrolytic activity. FIG. 7B shows the transglycosylation activity of the mutants. Compared with the wild-type, the transglycosylation activities of R176Y and D179A towards monoclonal antibody 2 were significantly increased. In summary, the mutants R176Y and D179A retain the majority of the hydrolytic activity in the activity assays of monoclonal antibody 1 and monoclonal antibody 2, while having significantly enhanced transglycosylation activity, indicating that the preferred mutants of the present disclosure exhibit universality for antibody substrates.

The transglycosylation product accumulation experiment for the preferred mutants R176Y and D179A against monoclonal antibody 1 was then conducted, with the results shown in FIG. 8. Both mutants R176Y and D179A efficiently accumulated the transglycosylation products, with more than 80% of the antibody glycosylated within 4 h. At the same time, after 20 h of reaction, the sample still exhibited 100% transglycosylated antibody 1 (FIG. 9), indicating that the retained hydrolytic activity of R176Y and D179A did not affect the stability of the antibody after glycoform remodeling, and can achieve the generation of 100% transglycosylated antibody products. Therefore, the mutants R176Y and D179A, possessing both glycosidic hydrolytic activity and transglycosylation activity, do not affect their application in the field of transglycosylation, and they can carry out the two steps of antibody site-specific glycoconjugation: antibody deglycosylation and sugar chain linker conjugation.

### Example 4: One-step Glycoform Remodeling of Intact Antibodies Directly by Wild-type Endo S2 and Its Mutants

In the foregoing Example, the glycoform remodeling of monoclonal antibody 1 was carried out in two steps. First, the antibody was deglycosylated by using the wild-type Endo S2 or its mutants. Then glycoside hydrolase was used to catalyze the conjugation of N3-LacNAc-Oxa. The above two-step method can prepare antibodies with bio-orthogonal reactive groups for ADC drugs. However, after deglycosylation, purification of the deglycosylated antibody is required, which makes the operation complex and the process cumbersome. Therefore, the R176Y and D179A mutants screened in the present disclosure were used to attempt one-step glycoform remodeling of intact mAbs (i.e., antibody deglycosylation and sugar chain linker conjugation were accomplished in one step). Meanwhile, the currently known best glycosynthases, the Endo S2 T138Q mutant and D184M mutant, were used as controls for the one-step method (see patent applications CN110234341A and CN109071630A for the T138Q mutant and D184M mutant).

The results of the one-step glycoform remodeling of intact monoclonal antibody 1 are shown in FIG. 10. FIG. 10A shows the control group without the addition of endoglycosidase. The ESI-MS deconvolution data of monoclonal antibody 1 heavy chain show that the antibodies in the system are all in their original glycoforms, i.e., G0 (50448±2 Da), G0F (49922 ±2 Da), G1F (50756±2 Da), and G2F (50918±2 Da) glycoforms. In the experimental systems of Endo S2 T138Q (FIG. 10B) and D184M (FIG. 10C), the major substances are m/z 49352±2 Da and 49499±2 Da, corresponding to the deglycosylated GlcNAc-monoclonal antibody 1 and Fucα1,6-GlcNAc-monoclonal antibody 1, accounting for more than 88%. While the glycoform remodeling products, GlcNAc-(N3-LacNAc)-monoclonal antibody 1 (m/z 49777±2 Da corresponding to azide shedding under acidic conditions, m/z 49801±2 Da corresponding to azide not shed) and Fucα1,6-GlcNAc-(N3-LacNAc)-monoclonal antibody 1 (m/z 49922±2 Da corresponding to azide shedding under acidic conditions, m/z 49946±2 Da corresponding to azide not shed), account for less than 12%. In the experimental systems of mutants R176Y (FIG. 10D) and D179A (FIG. 10E) of the present disclosure, the ESI-MS spectra mainly show substances with m/z 49801±2 Da, 49922 ±2 Da and 49946±2 Da, corresponding to the molecular weights of GlcNAc-(N3-LacNAc)-monoclonal antibody 1 (azide unshedded), Fucα1,6-GlcNAc-(N3-LacNAc)-monoclonal antibody 1 (azide shed under acidic conditions), and Fucα1,6-GlcNAc-(N3-LacNAc)-monoclonal antibody 1 (azide unshedded), respectively. Monoclonal antibody 1 essentially (more than 95%) completed the conjugation of the disaccharide linker, and the conjugation was homogeneous (DAR=2).

The results of the one-step glycoform remodeling of intact monoclonal antibody 2 are shown in FIG. 11. FIG. 11A shows the control group without the addition of endoglycosidase. The ESI-MS deconvolution data of the monoclonal antibody 2 heavy chain show that the antibodies in the system are in their original glycoforms, i.e., G0 (50379±2 Da), G0F (50525±2 Da), and G1F (50687±2 Da) glycoforms. In the experimental systems of Endo S2 T138Q mutant (FIG. 11B) and D184M mutant (FIG. 11C), the deglycosylated monoclonal antibody 2, including GlcNAc-monoclonal antibody 2 (m/z 49285±2 Da) and Fucα1,6-GlcNAc-monoclonal antibody 2 (m/z 49431±2 Da), accounted for more than 84%. In the experimental systems of R176Y mutant (FIG. 11D) and D179A mutant (FIG. 11E) of the present disclosure, more than 97% of the antibodies are glycoform remodeling products with homogeneous conjugation (DAR=2), including GlcNAc-(N3-LacNAc)-monoclonal antibody 2 (m/z 49707 ±2 Da corresponding to azide shedding under acidic conditions; m/z 49371±2 Da corresponding to azide not shed) and Fucα1,6-GlcNAc-(N3-LacNAc)-monoclonal antibody 2 (m/z 49852 ±2 Da corresponding to azide shedding under acidic conditions; m/z 49877±2 Da corresponding to azide not shed).

The data above indicate that the mutants R176Y and D179A can simultaneously complete two steps of antibody site-specific glycoconjugation in a one-step reaction: antibody deglycosylation and sugar chain linker conjugation. Moreover, they can prepare antibodies with bioorthogonal reaction groups more quickly than the best-known glycosidic synthetases, such as the Endo S2 T138Q mutant and the D184M mutant, thereby reducing production steps and lowering production costs.

The present disclosure describes mutant enzymes derived from the endo β-N-acetamidoglucosidase (Endo S2) of serotype M49 *Streptococcus pyogenes* NZ131 strain, which include mutation sites R176Y and/or D179A, and which, compared to the previously reported wild-type Endo S2 and its mutants, retain more glycosidic hydrolytic activity while exhibiting enhanced transglycosylation activity. Therefore, the mutant enzymes of the present disclosure have a broader range of application in antibody site-specific glycoconjugation processes, such as deglycosylated antibody production and sugar chain linker conjugation.

It will be appreciated by those of ordinary skill in the art to which the present disclosure pertains that the Examples above are illustrative only and that various changes and modifications may be made to mutant proteins without departing from the scope of the present disclosure. These changes and modifications include, but not limited to, insertion and/or truncation at the N-terminus and/or C-terminus, protein glycosylation, sialylation, albumination, farnesylation, carboxylation, hydroxylation, phosphorylation, conjugation to a polymer, and the like. What is important is that it possesses the Endo S2 catalytic domain and includes mutations at positions R176 and/or D179. Additionally, the Examples described herein utilize monoclonal antibodies, and those of ordinary skill in the art will understand that similar methods can be applied to other glycoproteins or antibodies. Furthermore, the Examples described herein utilize azide-modified LacNAc disaccharide linkages. Those skilled in the art will recognize that linker modification methods may also include, but are not limited to, sulfhydryl, alkynyl, and aldehyde modifications, and sugar chain linkers may also be extended to di-, tri-, tetra-, penta-, hexa-, hepta-, octa-, nona-, deca-, undecasaccharide, or other glycan forms.

## Claims

1. An Endo S2 mutant enzyme, which has a mutation at one or two of the following amino acid positions: R176 and/or D179, on the basis of:
(a) a sequence set forth in SEQ ID NO: 1 or an amino acid sequence having at least 95% sequence identity to SEQ ID NO: 1; or
(b) a sequence comprising contiguous amino acids from positions 38 to 819 of SEQ ID NO: 1,
wherein the mutant enzyme possesses glycosidic hydrolysis and/or transglycosylation activity.

2. The Endo S2 mutant enzyme according to claim 1, wherein the mutation is selected from at least one of: R176Y, and D179A.

3. A polynucleotide encoding the Endo S2 mutant enzyme according to claim 1 or 2.

4. A vector comprising the polynucleotide according to claim 3.

5. A host cell transformed with the vector according to claim 4.

6. Use of the Endo S2 mutant enzyme according to claim 1 or 2 in the glycol-engineering of an N-glycan-containing polypeptide or protein.

7. The use according to claim 6, which is used for the glycoform remodeling of an antibody, or for the sugar chain reconstruction of a glycoprotein, or for the preparation of a detection drug or ADC drug comprising an antibody.

8. A method for sugar chain remodeling of an N-glycan-containing polypeptide or protein using a one-step process, comprising the steps of:
(a) introducing the Endo S2 mutant enzyme according to claim 1 or 2;
(b) introducing the N-glycan-containing polypeptide or protein as a substrate;
(c) providing a sugar chain linker donor; and
(d) using the Endo S2 mutant enzyme to perform glycosidic hydrolysis on the N-glycan-containing polypeptide or protein and transfer the sugar chain linker donor to the glycosidic hydrolysis product, thereby providing a new sugar chain-engineered polypeptide or protein;
wherein the one-step process does not comprise a purification step for the glycosidic hydrolysis product.

9. A method for sugar chain remodeling of an N-glycan-containing polypeptide or protein using a two-step process, wherein two enzymes are introduced to perform glycosidic hydrolysis and new glycosyl transfer, respectively, wherein the Endo S2 mutant enzyme according to claim 1 or 2 is used in at least one step of glycosidic hydrolysis or new glycosyl transfer, the method comprising the following steps:
(a) introducing the Endo S2 mutant enzyme according to claim 1 or 2 or other suitable endoglycosidases;
(b) introducing an N-glycan-containing polypeptide or protein as a substrate, and performing glycosidic hydrolysis under the action of the enzyme in step (a), thereby providing a glycosidic hydrolysis product as an acceptor for the subsequent new glycosyl transfer;
(c) providing a sugar chain linker donor; and
(d) transferring the sugar chain linker donor to the acceptor by the Endo S2 mutant enzyme or other suitable glycosyltransferases, to generate a new sugar chain-engineered polypeptide or protein;
optionally, a purification step for the glycosidic hydrolysis product is also comprised between steps (b) and (c).

10. The method according to claim 8 or 9, wherein the N-glycan-containing polypeptide or protein comprises naturally occurring antibodies, recombinant antibodies, Fc fragments of antibodies, or sialoglycopeptides.

11. The method according to claim 8 or 9, wherein the sugar chain linker donor is a synthetic glycan oxazoline or a natural N-glycan oxazoline.

12. The method according to claim 11, wherein the natural N-glycan oxazoline comprises complex glycan oxazoline, high-mannose glycan oxazoline, or hybrid oxazoline.

13. The method according to claim 11, wherein the synthetic glycan oxazoline is a disaccharide oxazoline, a trisaccharide oxazoline, a tetrasaccharide oxazoline, a pentasaccharide oxazoline, a hexasaccharide oxazoline, a heptasaccharide oxazoline, an octasaccharide oxazoline, a nonasaccharide oxazoline, a decasaccharide oxazoline, or an undecasaccharide oxazoline.

14. The method according to claim 8 or 9, wherein the sugar chain linker donor further comprises an additional modification method or an additional tag.

15. The method according to claim 14, wherein the additional modification method is selected from azidation, aldehydation, alkynylation, sulfhydrylation, hydroxylation, carboxylation, phosphorylation, sialylation, albumination, farnesylation, or conjugation to a polymer.

16. The method according to claim 14, wherein the additional tag is selected from therapeutic drugs, toxin substances, fluorescent probes, biotin, lipids, antigen fragments, PEG, RNA, DNA, polypeptides, proteins, enzymes, enzyme substrates, enzyme inhibitors, enzyme modulators, or antibody fragments.

17. A sugar chain-remodeled antibody and an ADC drug thereof, prepared by the method according to any one of claims 8-16.
